(19) Europäisches Patentamt
European Patent Office
Office européen des brevets

(11) Veröffentlichungsnummer : **0 023 040**
**B1**

(12) **EUROPÄISCHE PATENTSCHRIFT**

(45) Veröffentlichungstag der Patentschrift : **14.09.83**

(51) Int. Cl.³ : **C 02 F 1/76**, A 61 L 2/20, A 61 L 9/015

(21) Anmeldenummer : **80104241.7**

(22) Anmeldetag : **18.07.80**

(54) **Vorrichtung zur Entkeimung von strömungsfähigen Mitteln.**

(30) Priorität : **23.07.79 DE 2929813**

(43) Veröffentlichungstag der Anmeldung : **28.01.81 Patentblatt 81/04**

(45) Bekanntmachung des Hinweises auf die Patenterteilung : **14.09.83 Patentblatt 83/37**

(84) Benannte Vertragsstaaten : **AT BE CH DE FR GB IT LI LU NL SE**

(56) Entgegenhaltungen :
**CA A 965 699**
**FR A 814 972**
**FR A 2 230 592**
**FR A 2 345 393**
**GB A 1 233 418**
**US A 3 412 935**
**US A 3 647 523**

(73) Patentinhaber : **Hoelzle & Chelius GmbH**
**Hugenottenallee 150**
**D-6078 Neu Isenburg (DE)**

(72) Erfinder : **Alvaro, Affonso, Prof. Dr.**
**Rind'sche-Stift-Strasse 2**
**D-6380 Bad Homburg v. d. H. (DE)**

(74) Vertreter : **Linser, Heinz et al**
**Patentanwälte Heinz Linser Dipl. Ing. Eckhardt Eyer**
**Robert-Bosch-Strasse 12a Postfach 10 22 10**
**D-6072 Dreieich (DE)**

**EP 0 023 040 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Vorrichtung zur Entkeimung von strömungsfähigen Mitteln

## Beschreibung

Die Erfindung betrifft eine Vorrichtung zur Entkeimung von strömungsfähigen Mitteln, insbesondere Wasser oder Luft, mit Hilfe eines Stoffes, der bei Zugabe eines Aktivators in Form einer wäßrigen Lösung ein desinfizierend wirkendes Gas entwickelt.

Auf dem Gebiet der Sterilisation von medizinischen Instrumenten oder beispielsweise der Haltbarmachung von Lebensmitteln ist es bekannt, Kunststoff-Folien zu verwenden, welche für gasförmige Stoffe durchlässig sind. So ist es insbesondere für die Verpackung von Lebensmitteln wichtig, die Sauerstoffdiffusion in Richtung der verpackten Lebensmittel und die Diffusion von Kohlendioxid und aromatischen Gasen von den verpackten Lebensmitteln in die Atmosphäre zu vermindern. Die Diffusion der Gase und der aromatischen Stoffe durch die Kunstoff-Folie werden von vielen Faktoren beeinflußt, wobei die wichtigsten in der Natur der Gase, der Temperatur und des Druckes sowie der Eigenschaften der Kunststoff-Folie bestehen.

Die Gasdiffusion durch Kunststoff-Folien ist in gleicher Weise zu deuten wie die Diffusion bei Flüssigkeiten, nämlich das Gas löst sich in dem Kunststoffmaterial, wandert hindurch und tritt schließlich auf der anderen Folienseite als Gas aus, wo es gegebenenfalls wieder in Lösung mit dem dort vorhandenen Mittel geht.

Die verpackungstechnologisch sehr wichtige Aromadichtigkeit verläuf mit den Sperreigenschaften für Gase nicht parallel und ist sehr stark vom chemischen Aufbau des Aromastoffes abhängig. Bei Folien, die Feuchtigkeit aufnehmen, wie beispielsweise Zellglas und Polyamid, wird die Diffusion mit erhöhtem Feuchtegrad beschleunigt. Die Diffundierbarkeit ist dabei nicht von der Foliendicke abhängig, sondern ausschließlich vom Material. Die Foliendicke stellt lediglich einen Zeitfaktor dar, hat jedoch einen starken Einfluß auf die Diffusionswerte.

Die Sterilisation von Trinkwasser oder Wasser in Badeanstalten wird zur Zeit überwiegend mit Chlorgas aus Gasflaschen durchgeführt. Die Herstellung von Chlorgas, seine Einfüllung in die Flaschen, der Transport, die Lagerung und das Entleeren bei der Sterilisation sind aufwendig, nicht ungefährlich, sodaß die Handhabung nur durch Fachleute erfolgen darf, und schließlich kompliziert. So ist insbesondere bei der Sterilisation eine äußerst genaue Dosierung einzuhalten, um eine Gefährdung von Personen zu vermeiden. Aufgrund dieser Tatsache wurden auch bereits Versuche unternommen, chemische Verbindungen einzusetzen, welche Chlorgas erzeugen. Ein erheblicher Nachteil des Einsatzes derartiger chlorerzeugender Stoffe besteht jedoch darin, daß nach der Gasabgabe die Reststoffe der chemischen Verbindungen im zu sterilisierenden Wasser verbleichen, sodaß eine unerwünschte oder sogar toxische Wirkung derselben nicht ausgeschlossen ist. Dies bedeutet, daß beispielsweise bei der Sterilisation des Wassers im Schwimmbecken der Schwimmende auch mit diesen Reststoffen in Berührung kommt, wobei auch eine innere Anwendung nicht ausgeschlossen werden kann.

Da die gasabgebenden Stoffe einer technischen Produktion entstammen, sind darüberhinaus auch Verunreinigungen enthalten, und selbst bei einer Unschädlichkeit des verbleibenden Reststoffes sind schädliche oder unerwünschte Verunreinigungen nicht ausgeschlossen, da die Produkte nicht analysenrein hergestellt werden.

Aus der deutschen Offenlegungsschrift 17 67 635 geht ein Chlorfilter hervor, welcher insbesondere für Schwimmbecken verwendet werden soll. Hierbei werden die Chlortabletten bzw. Chlorgranulate zunächst in der richtigen Dosierung in einen Beutel aus Kunstoff verpackt, der über seine Fläche bzw. über einen Teil seiner Fläche perforiert ist. Für den Transport oder die Aufbewahrung der Chlortabletten wird dieser perforierte Kunststoffbeutel in einen zweiten Kunstoffbeutel verpackt und absolut dicht verschlossen, sodaß keine Chlorgase ausströmen können. Ein mit einer Perforation versehener Kunststoffbeutel ist jedoch nicht geeignet, Verunreinigungen der chlorgaserzeugenden Stoffe zurückzuhalten.

Aus der deutschen Offenlegungsschrift 16 42 474 geht weiter ein Verfahren und eine Vorrichtung zum Regenerieren von Wasser mit gasförmigen Medien hervor, wobei das Gas, wie beispielsweise Kohlendioxid, mit der Flüssigkeit durch Diffusion vermischt werden soll. Zur Durchführung dieses Verfahrens wird eine Vorrichtung verwendet, die aus einem teilweise mit Gas zu füllenden Behälter besteht, der einen unmittelbaren Kontakt zwischen Gas und Flüssigkeit herstellt, um somit güngstige Diffusionsmöglichkeiten zu schaffen. Um die Diffusion auf einen bestimmten Bereich zu beschränken und im Behälter stets einen gewissen Gasvorrat aufrechtzuerhalten, werden gasdurchlässige Trennwände oder Siebe vorgesehen. Diese Mittel dienen somit dazu, den Vermischungsvorgang zu verlangsamen, um einen stetigen Prozeß aufrechterhalten zu können.

Aus der FR-A-2 230 592 geht eine Vorrichtung zur Entkeimung von strömungsfähigen Mitteln mit Hilfe eines Stoffes hervor der bei Zugabe eines Aktivators ein desinfizierendes Gas entwickelt.

Diese bekannte Vorrichtung ist jedoch wirkungslos, wenn der Behälter mit dem Chlordioxyd fest verschlossen ist, da der Behälter eine Öffnung benötigt, aus der das Chlordioxyd und die Reaktionsprodukte gelangen können.

Aus der USA-A-3 412 935 ist ein System bekannt, mit dessen Hilfe die gesteuerte Einführung eines

Gases in ein flüssiges Medium durchgeführt werden soll. Das verwendete Gas befindet sich hierbei unter hohem Druck, bei dem gleichzeitig seine flüssige Phase anwesend ist. Aus diesen Gründen ist ein druckfestes Gefäß aus gaspermeablem Kunststoff erforderlich, das mit einem Druckreduzierventil zu versehen ist. Dieses bekannte System ist daher bei normalem Atmosphärendruck nicht arbeitsfähig.

Der vorliegenden Erfindung liegt die Aufgabe zugrunde, eine Vorrichtung zur Entkeimung von strömungsfähigen Mitteln, insbesondere von Wasser oder Luft vorzuschlagen, mit deren Hilfe ein desinfizierendes Gas aus einem Stoff erzeugt wird, dessen Restprodukt einschließlich seiner Verunreinigungen nicht mit dem zu desinfizierenden Mittel in Berührung kommt.

Der Erfindung liegt weiter die Aufgabe zugrunde, eine Gasquelle vorzuschlagen, welche unter normalen Bedingungen, beispielsweise bei Zimmertemperatur und Normal-Luftdruck kontinuierlich arbeitet, wobei die Steuerung der Gaserzeugung möglich ist.

Die Lösung dieser Aufgabe erfolgt mit der eingangs aufgeführten Vorrichtung dadurch, daß sich der Stoff in einem Behälter befindet, welcher mindestens eine Seitenwandung aus einer gasdurchdiffundierbaren Kunststoff-Folie aufweist, welche gleichzeitig das Restprodukt des Stoffes einschließlich seiner Verunreinigungen zurückhält, und daß die Außenseite der Kunststoff-Folie mit dem zu entkeimenden strömungsfähigen Mittel in Kontakt steht.

In Weiterbildung der Erfindung befindet sich der bei der Zugabe eines Aktivators desinfizierende, gasentwickelnde Stoff in einem Beutel, Schlauch oder einer Patrone aus Kunststoff, wobei der Beutel, Schlauch oder die Patrone eine der Zuführung des Aktivators dienende Einrichtung aufweist und der/die Beutel oder Schlauch/Schläuche oder Patrone(n) in einem Gefäß angeordnet ist/sind, welches von dem zu entkeimenden Mittel durchströmt wird.

Als Kunststoff-Folie wird nach der Erfindung eine Polyäthylenfolie (PE) weich oder hart, eine Polypropylen- (PP), Polyvinylchlorid- (PVC), Weich-PVC-, Polyäthylenpolypropylenpolyamid-, Polytetrafluoräthylen-Folie oder eine Folie aus Copolymeren und/oder Abkömmlingen dieser Kunststoffgruppen verwendet.

Das desinfizierende Gas besteht vorzugsweise aus Halogen, seinen gasförmigen Abkömmlingen oder Verbindungen, beispielsweise den Oxiden oder aus Schwefeldioxid. Der Behälter, Beutel, Schlauch oder die Patrone kann in Weiterbildung der Erfindung aus zwei Kammern bestehen, wobei die eine den bei Zugabe eines Aktivators ein desinfizierendes Gas entwickelnden Stoff (Gasspender) und die andere den Aktivator enthält, wobei die Kammern durch eine auslösbare Trennvorrichtung voneinander getrennt sind.

Der Vorteil der Vorrichtung nach der Erfindung besteht darin, daß nunmehr ein gaserzeugender Stoff verwendet werden kann, der in einer technischen Produktion preiswert hergestellt wird, d. h., welcher nicht analysenrein ist und als Restprodukt unerwünschte oder toxische Eigenschaften aufweisen kann.

In einer Ausführungsform der Erfindung strömt das zu entkeimende Mittel entlang der Seite der Folienoberfläche, durch die das desinfizierende Gas diffundiert ist, sodaß das Gas kontinuierlich abgeführt wird. Auf diese Weise läßt sich ein chemisches Ungleichgewicht in Richtung der Gaserzeugung fortlaufend aufrechterhalten. Durch die Strömungsgeschwindigkeit des zu entkeimenden Mittels läßt sich neben der Zugabe eines bestimmten Aktivators die Gaserzeugung steuern.

In einer Weiterbildung der Erfindung ist es auch möglich, den ein desinfizierendes Gas entwickelnden Stoff kontinuierlich durch eine Austauscheinrichtung zu führen. Hierbei kann der das desinfizierende Gas spendende Stoff sich in einem Vorratsbehälter befinden, welcher strömungsmäßig mit der Austauschvorrichtung verbunden ist, wobei zur Auslösung der chemischen Reaktion dem Vorratsbehälter eine Flüssigkeit, beispielsweise Wasser, zugeführt wird. Die Durchflußgeschwindigkeit wird dabei derartig geregelt, daß der gasspendende Stoff nahezu vollständig das desinfizierende Gas abgibt.

Die Erfindung wird anhand der Zeichnung näher erläutert.

Hierbei zeigen :

Figur 1 einen Beutel aus Polyäthylen, welcher luft- und wasserdicht verschweißt ist ;

Figur 2 einen Beutel ähnlich wie in Figur 1, jedoch mit getrennten Kammern ;

Figur 3 eine Dosierpatrone mit dicht verschließbarer Einfüllöffnung ;

Figur 4 einen Durchlauf-Dosierbehälter ;

Figur 5 eine weitere Variante einer Durchflußdosiereinrichtung und die

Figuren 6-11 Anwendungsbeispiele der Erfindung.

Figur 1 zeigt einen Beutel 2 aus Polyäthylen, der luft- und wasserdicht verschweißt ist und in dem sich der Stoff 1 befindet, der desinfizierendes Gas entwickelt. Dieser Stoff 1 kann beispielsweise aus dem Natriumsalz der Dichlorisocyanursäure mit der chemischen Formel $C_3N_3O_3Cl_2Na$ bestehen. Ferner befindet sich darin der Aktivator 3, eine Flüssigkeit mit einem definierten pH-Wert.

Die desinfizierende Eigenschaft dieser und anderer chlorierter Isocyanursäuren entsteht durch die Freigabe aktiver Chlorgase bei der Zugabe von Wasser. Als Ergebnis dieser Hydrolyse entsteht Isocyanursäure.

Als Gasspender lassen sich sowohl organische als auch anorganische Abspalter verwenden. Als organische Chlorabspalter eignen sich Chloramin B (Benzolsulfochloramin-Na), ferner Chloramin T, welches in Wasser naszierenden Sauerstoff entwickelt. Zur Trinkwasser-Desinfektion lassen sich p-Dichlorsulfamylbenzoesäure verwenden. Als anorganische Halogenspender, insbesondere Chlorspender, laßt sich beispielsweise Natriumchlorit verwenden, welches in einer sauren Lösung beispielsweise unter Verwendung von Amidosulfonsäure Chlordioxid entwickelt, nach folgender Beziehung :

$$5\ Na\,ClO_2 + HSO_3NH_2 \rightarrow 4\ ClO_2 + 4\ NaSO_3NH_2 + NaCl + 2\ H_2O.$$

Die Figur 2 zeigt einen Beutel ähnlich der Figur 1, welcher aus zwei Kammern 8 und 9 besteht, welche voneinander durch eine auslösbare Trennvorrichtung 10 getrennt sind. In der oberen Kammer 8 befindet sich der Aktivator 3 und in der unteren Kammer 9 der Gasspender 1. Die auslösbare Trennvorrichtung 10 besteht bei diesem Ausführungsbeispiel aus einem Klipp, nach dessen Auslösung der Aktivator mit dem Gasspender in Kontakt kommt. Unmittelbar danach erfolgt die Erzeugung des entsprechenden Gases, welches durch den Beutel nach außen diffundiert.

Die Figur 3 zeigt eine Dosierpatrone 4 mit dicht verschließbarer Einfüllöffnung. Die Umhüllung 11 der Patrone besteht dabei aus einer Polyäthylenfolie mit einem Stützgewebe. Die Einfüllöffnung 12 läßt sich mit der Hülle der Dosierpatrone dicht verschliessen.

Der Boden 13 der Dosierpatrone ist mit der Seitenwandung fest verschweißt und im unteren Teil der Patrone befindet sich der Gasspender 1. Dieser wird ausgelöst, wenn durch die Einfüllöffnung 12 der Aktivator, beispielsweise Wasser eingefüllt wird.

Die Figur 4 zeigt einen Durchlaufdosierbehälter 14, welcher mit Dosierpatronen 15 der in Figur 3 dargestellten Art ausgerüstet ist. Im oberen Teil des Dosierbehälters 14 ist eine Öffnung 16 zum Wechsel der Patrone 15 vorgesehen. Ein Entlüftungsventil 17 dient zum Druckausgleich des Innenraumes des Behälters 14. Im Bodenteil ist ein Zulauf 18 und im Deckelteil ein Ablauf 19 vorhanden.

Die Patronen 15 werden nach Aktivierung von dem zu desinfizierenden Stoff umspült, sodaß ein quasi kontinuierlicher Betrieb durchführbar ist.

In Figur 5 ist ein weiteres Ausführungsbeispiel der Erfindung dargestellt. Bei der in Figur 5 dargestellten Durchflußdosierungseinrichtung befinden sich in dem Behälter 20 mit einem Abfluß 24 schlauchartige Gefäße 21 aus Polyäthylen, gegebenenfalls mit einem Stützgewebe, welche nebeneinander angeordnet und hintereinander geschaltet sind. Damit läßt sich auch der das desinfizierende Gas entwickelnde Stoff mit seinem Aktivator kontinuierlich durch die Gefäße 21 führen, sodaß ein vollständiger kontinuierlicher Betrieb möglich ist. Durch den Zufluß 23 strömt das zu desinfizierende Mittel ein und umspült dabei sämtliche Gefäße 21, durch deren Wandungen das erzeugte Gas diffundiert. Das Gas geht in dem zu desinfizierenden Mittel in Lösung und wird sogleich abgeführt, sodaß die Diffusion durch die Wandung der Gefäße 21 gleichmäßig aufrechterhalten wird.

Die Entwicklung der Gaserzeugung im Innern der Gefäße 21 läßt sicht durch Veränderung des pH-Wertes steuern, je nach dem, welch ein gaserzeugendes Mittel verwendet wird. Bevorzugt ist ein saurer Aktivator.

Durch Verwendung entsprechender Ionenaustauscher lassen sich die pH-Werte in an sich bekannter Weise verändern, sodaß die Reaktion bzw. die Gaserzeugung gesteuert werden kann.

Der das desinfizierende Gas entwickelnde Stoff wird mit seinem Aktivator dem Einlaßrohr 25 zugeführt, durchströmt die Gefäße 21 und verläßt diese wieder durch das Auslaßrohr 26.

Die Figur 6 zeigt ein einfaches Anwendungsbeispiel für die Entkeimung von kleinen Mengen Trinkwassers, beispielsweise in Katastrophengebieten. Das zu desinfizierende Wasser befindet sich in einem Gefäß 27, in das die Patrone 28 mit Hilfe eines Gestänges 29 getaucht wird. In der Patrone befindet sich der Chlorspender 30 mit seinem Aktivator. Die Patrone kann beispielsweise aus Polytetrafluoräthylen bestehen. Ein derartiges Polyäthylen ist besonders gegen Chemikalien beständig, so daß eine solche Patrone eine relativ lange Lebensdauer besitzt.

Die Figur 7 zeigt einen Wasserenthärter 31 mit einem Zuflußstutzen 32 und einem Abflußstutzen 33, welcher mit dem Rohr 36 verbunden ist. Das Rohr hat an seinem unteren Ende mehrere Öffnungen. Der Wasserenthärter 31 ist mit einem Ionenaustauscher 34 bis zu einer bestimmten Höhe gefüllt. Das Leitungswasser durchströmt die Ionenaustauscherzone, so daß der Ionenaustausch stattfinden kann, steigt danach durch das Rohr 36 und wird durch den Abflußstutzen 33 entnommen. In dem Rückspülraum über dem Ionenaustauscher befindet sich eine Patrone 28, die ebenfalls mit einem Chlorspender 30 und einem Aktivator gefüllt ist. Die Patrone 28 wird von einem Ballast 35 auf der Ionenaustauscher-Oberfläche gehalten. Das sich in dem Wasserenthärter 31 ansammelnde Leitungswasser wird durch den Chlorspender 30 desinfiziert und durchwandert den Ionenaustauscher 34 zur Enthärtung, gelangt sodann in die unteren Öffnungen des Rohres 36 und wird diesem über das Abflußrohr 33 enthärtet und desinfiziert entnommen. Das im Filtergefäß 31 befindliche, entkeimte Wasser hat somit gleichzeitig zur Folge, daß auch während Stillstandszeiten die Verkeimung des Ionenaustauschers verhindert wird.

Aus der Figur 8 geht ein anderes Anwendungsbeispiel hervor. Für die Entkeimung von Sanitäranlagen befindet sich eine Patrone 28 aus gasdurchlässigem Kunststoff, gefüllt mit ihrem Halogenspender und Aktivator in einem Toilettenspülkasten 37. Eine solche Maßnahme ist besonders in Krankenhäusern und Seuchenstationen vorteilhaft.

Die Erfindung eignet sich auch als Gärungsprozeßhemmer bei der Hefe-Entkeimung beispielsweise von Weinfässern aus Holz. Ein solches Ausführungsbeispiel geht aus Figur 9 hervor. In diesem Fall ist eine Patrone 28 mit einem Schwefeldioxidspender und einem Aktivator 38 gefüllt und befindet sich in einem mit Wasser gefüllten Weinfaß 39. Für diese Anwendungszwecke eignet sich Schwefeldioxid besser als Chlor, da evtl. in dem Weinfaß verbleibende Schwefelrückstände keine negativen Wirkungen auf die

Entwickung des Weines im Faß aufweisen.

Zur Reinigung von Flaschen, Milchkannen, Gläsern und dergleichen kann es vorteilhaft sein, einen Vorratsbehälter mit desinfizierendem Spülwasser zur Verfügung zu haben. Ein solches Ausführungsbeispiel geht aus Figur 10 hervor. Eine Batterie von Patronen 28 aus gasdurchlässigem Kunststoff, gefüllt mit einem Chlorspender und Aktivator 30 befindet sich in einem Vorratsbehälter 40. Die Patronen 28 werden von einem Stützgitter 41 gehalten, welches in den Vorratsbehälter 40 eingehängt werden kann. Mit Hilfe einer Pumpe wird das zu desinfizierende Wasser in den Tank 40 gepumpt und wird bei 42 dem Tank desinfiziert entnommen.

Ein weiteres Problem besteht bei der Lagerung von Trinkwasser. Bereits nach wenigen Tagen haben sich in einem Wassertank Algen und Bakterien derartig vermehrt, daß es ungenießbar wird. Schon nach einer Lagerung von 5-8 Tagen entsteht eine Geschmacksänderung, die sich bis zur Ungenießbarkeit weiterentwickelt. Ein solches Problem tritt beispielsweise bei in See gehenden Yachten auf, welche nicht mit komplizierten Aufbereitungsanlagen ausgerüstet sind. Weitere entsprechende Anwendungsfälle dieser Art liegen auf der Hand. Hierfür gibt Figur 11 ein Ausführungsbeispiel an, bei dem eine Batterie von Patronen 28, welche mit einem Chlorspender und Aktivator 30 gefüllt sind, sich in einem Trinkwasserreservoir 46 befinden. Die Patronen 28 werden von einem Gerüst 44 in ihrer Lage fixiert, welches durch eine schwere Masse 45 auf dem Grund des Trinkwasserreservoires gehalten wird.

Mit der Erfindung wird der große Vorteil erreicht, daß zur Erzeugung eines desinfizierenden Gases Stoffe verwendet werden können, deren Restbestandteile unerwünschte oder sogar toxische Wirkungen zeigen können. Diese Stoffe werden durch die Folie bzw. Patronenwandung zurückgehalten, sodaß jegliche unerwünschte oder toxische Wirkungen bei dem zu desinfizierenden Mittel im Humanbereich ausgeschlossen sind. Damit lassen sich Gaserzeuger einsetzen, welche preiswert herzustellen sind und aufgrund ihrer Zusammensetzungen sowie der damit resultierenden unerwünschten oder teilweise toxischen Wirkungen im Humanbereich bisher nicht eingesetzt werden konnten.

**Ansprüche**

1. Vorrichtung zur Entkeimung von strömungsfähigen Mitteln, insbesondere Wasser oder Luft, mit Hilfe eines Stoffes, der bei Zugabe eines Aktivators in Form einer wäßrigen Lösung ein desinfizierendes Gas entwickelt, dadurch gekennzeichnet, daß sich der Stoff in einem Behälter befindet, welcher mindestens eine Seitenwandung aus einer gasdurchdiffundierbaren Kunststoff-Folie aufweist, welche gleichzeitig das Restprodukt des Stoffes einschließlich seiner Verunreinigungen zurückhält, und daß die Außenseite der Kunststoff-Folie mit dem zu entkeimenden strömungsfähigen Mittel in Kontakt steht.

2. Vorrichtung nach Anspruch 1, dadurch gekennzeichnet, daß

a) der bei der Zugabe eines Aktivators ein desinfizierendes Gas entwickelnde Stoff sich in einem Beutel (2), einem Schlauch (3) oder einer Patrone aus Kunststoff befindet,

b) der Beutel, der Schlauch oder die Patrone eine der Zuführung des Aktivators dienende Einrichtung aufweist und

c) der/die Beutel oder Schlauch/Schläuche oder Patrone(n) in einem Gefäß (4) angeordnet ist/sind, welches von dem zu entkeimenden Mittel (5) durchströmt wird.

3. Vorrichtung nach Anspruch 1 oder 2, dadurch gekennzeichnet, daß als Kunstoff-Folie eine Polyäthylenfolie (PE) weich oder hart, eine Polypropylen- (PP), Polyvinylchlorid- (PVC), Weich-PVC-, Polyäthylenpolypropylenpolyamid-, Polytetrafluoräthylen-Folie oder eine Folie aus Copolymeren und/oder Abkömmlingen dieser Kunststoffgruppen verwendet wird.

4. Vorrichtung nach Anspruch 1, 2 oder 3, dadurch gekennzeichnet, daß der bei Zugabe eines Aktivators ein desinfizierendes Gas entwickelnde Stoff Halogen, Halogendioxid, Schwefeldioxid oder deren Derivate entwickeln kann.

5. Vorrichtung nach Anspruch 1 oder einem der voranstehenden, dadurch gekennzeichnet, daß der Behälter, Beutel, Schlauch oder die Patrone aus zwei Kammern besteht, wobei die eine Kammer den bei Zugabe eines Aktivators ein desinfizierendes Gas entwickelnden Stoff und die andere Kammer den Aktivator enthält und die Kammern durch eine aufhebbare Trennvorrichtung (10) voneinander getrennt sind.

6. Vorrichtung nach Anspruch 1 oder einem der voranstehenden, dadurch gekennzeichnet, daß Mittel zur Steuerung der Entwicklung des desinfizierenden Gases durch Veränderung des pH-Wertes der wäßrigen Lösung vorhanden sind.

**Claims**

1. An apparative set up for the sterilisation of fluids, specially water and air, comprising of a substance that, by the addition of an activator in aqueous solution, is capable of setting free a gas having desinfecting action, a container in which the said substance is enclosed, the container being characterised by the fact that, at least one side wall of it, is made of a plastic foil which, besides, being permeable to gas, is simultaneously impermeable and hence retains all residual products including

impurities. Furthermore the said side wall is in permanent contact with the fluid to be sterilised.

2. Set up according to claim 1 furthermore characterised by the following :

a) that the substance, which sets free the sterilising gas upon addition of an activator, is enclosed in 1. a bag, 2. a tube, 3. a cartridge made of plastic material.

b) That the bag, tube or cartridge permits the addition of the activator to its contents.

c) That the bag, tube or tubes or cartridge or cartridges are so place in a bigger container that the fluid to be sterilised can be made to flow past all of them.

3. Set up according to claims 1 and 2 characterised further by the fact that, the plastic foil is made of a polymer belonging to the group of polyethylene (soft or hard) polypropylene (PP) Polyvinylchloride (PVC), (Soft PVC) Polyethylene polypropylene polyamide, Polytetrafluoroethylene or a foil made of copolymers of above mentioned group of polymers and derivatives of the same.

4. Set up according to claims 1, 2, 3, characterised furthermore by the fact that the sterilising gas set free by the above mentioned substance, on addition of an activator, is a halogen, a halogen dioxide, sulphur dioxide or derivatives of the same.

5. Set up according to claim 1, or anyone mentioned above, characterised by the fact that the container namely bag, tube or cartridge is so constructed that two chambers are formed, one of wich contains the substance which sets free the desinfecting gas on addition of an aqueous solution of an activator, and the other, the activator solution itself. The separation wall between the two chambers can be removed (10).

6. Set up according to claim 1, or anyone of the above mentioned set ups, characterised by the fact that the regulation and formation of the desinfecting gas is achieved by the change of pH-value of the aqueous solution.

**Revendications**

1. Dispositif pour la stérilisation d'agents fluides, en particulier, de l'eau ou de l'air, à l'aide d'une substance qui dégage un gaz désinfectant lors de l'addition d'un activateur sous forme d'une solution aqueuse, caractérisé en ce que cette substance se trouve dans un récipient comportant au moins une paroi latérale réalisée en une feuille de matière synthétique à travers laquelle peut se diffuser un gaz et retenant en même temps le produit résiduaire de la substance, y compris ses impuretés, tandis que la face extérieure de la feuille de matière synthétique entre en contact avec l'agent fluide à stériliser.

2. Dispositif suivant la revendication 1, caractérisé en ce que :

a) la substance dégageant un gaz désinfectant lors de l'addition d'un activateur se trouve dans un sac (2), un tuyau (3) ou une cartouche en matière synthétique ;

b) le sac, le tuyau ou la cartouche comporte un dispositif assurant l'alimentation de l'activateur, et

c) le ou les sacs, le ou les tuyaux ou la ou les cartouches est ou sont disposé(s) dans un récipient (4) à travers lequel circule l'agent à stériliser (5).

3. Dispositif suivant la revendication 1 ou 2, caractérisé en ce que, comme feuille de matière synthétique, on utilise une feuille de polyéthylène molle ou dure, une feuille de polypropylène, de chlorure de polyvinyle, de chlorure de polyvinyle mou, de polyéthylène-polypropylène-polyamide, de polytétrafluoréthylène ou encore une feuille de copolymères et/ou de dérivés de ces groupes de matières synthétiques.

4. Dispositif suivant la revendication 1, 2 ou 3, caractérisé en ce que la substance dégageant un gaz désinfectant lors de l'addition d'un activateur peut dégager un halogène, un dioxyde d'halogène, de l'anhydride sulfureux ou leurs dérivés.

5. Dispositif suivant la revendication 1 ou une des revendications précédentes, caractérisé en ce que le récipient, le sac, la cartouche ou le tuyau est constitué de deux chambres, une chambre contenant la substance dégageant un gaz désinfectant lors de l'addition d'un activateur et l'autre chambre contenant l'activateur, ces chambres étant séparées l'une de l'autre par un dispositif de séparation amovible (10).

6. Dispositif suivant la revendication 1 ou une des revendications précédentes, caractérisé en ce qu'on prévoit des moyens en vue de régler le dégagement du gaz désinfectant en modifiant le pH de la solution aqueuse.

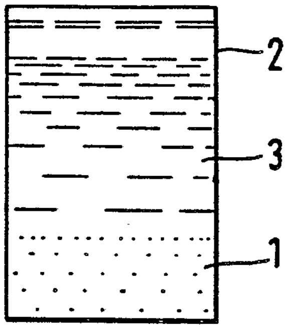


FIG. 1

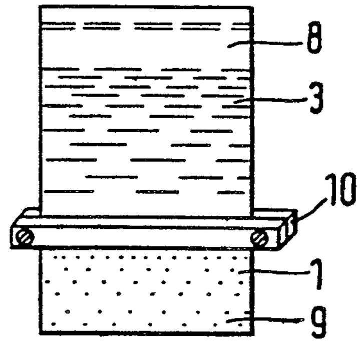


FIG. 2

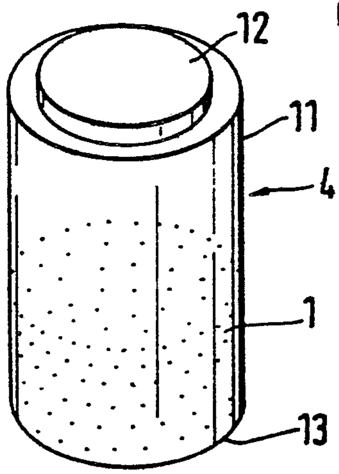


FIG. 3

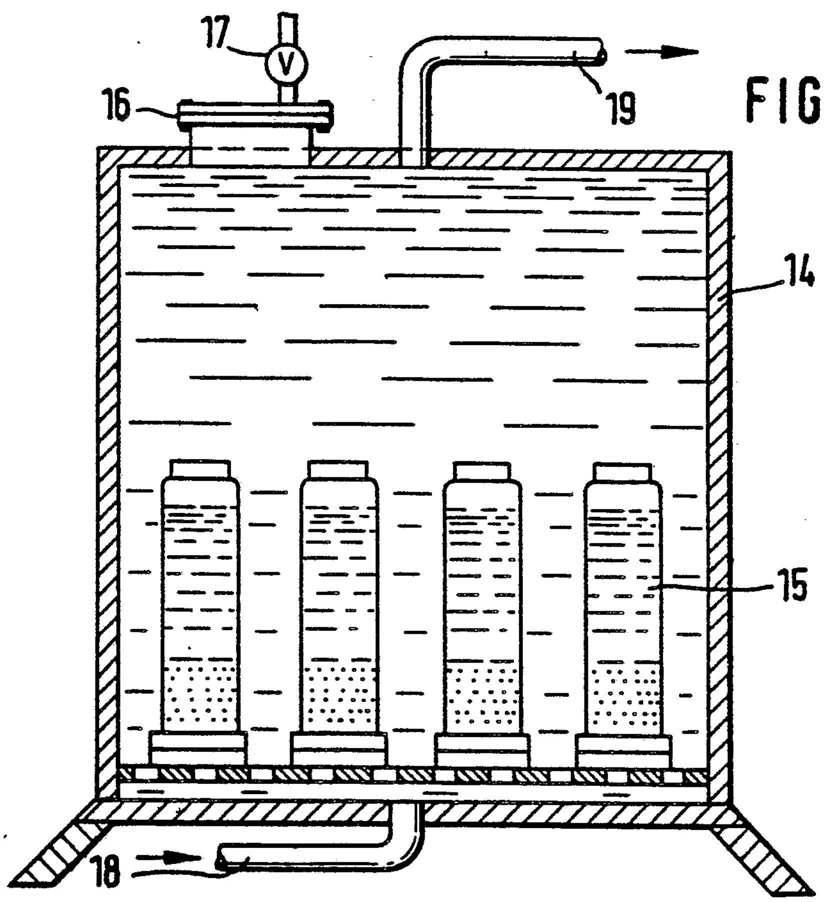


FIG. 4

FIG. 5

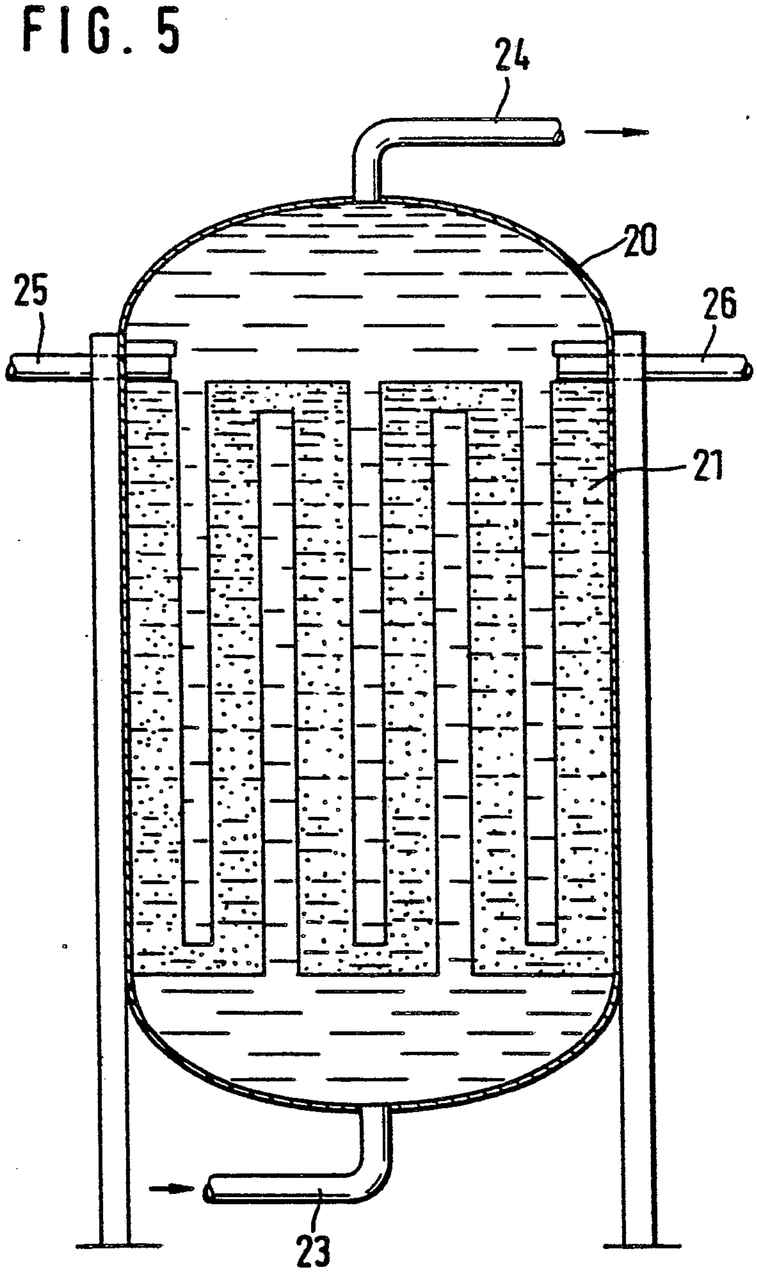

FIG. 6

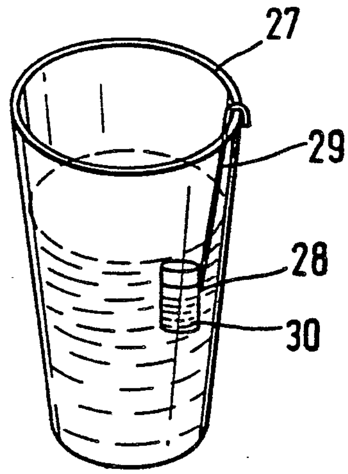


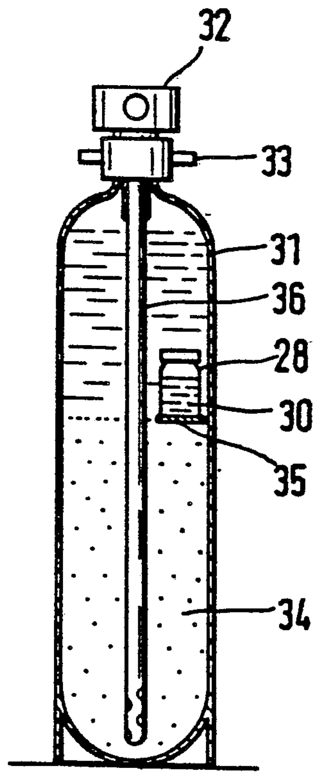


FIG. 7

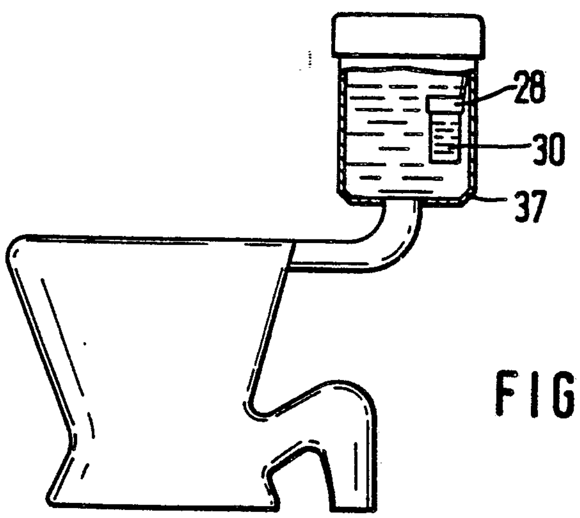


FIG. 8

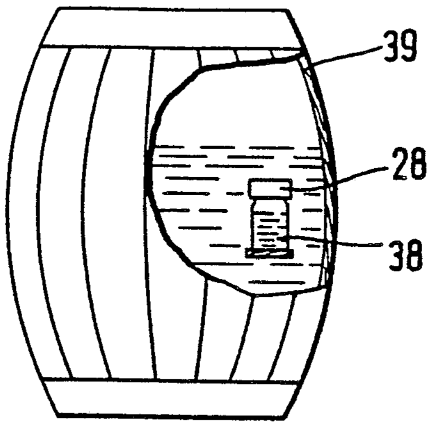


FIG. 9

FIG. 10

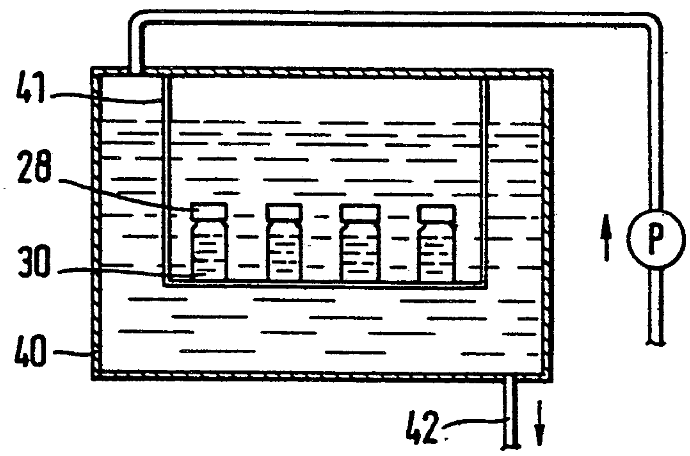


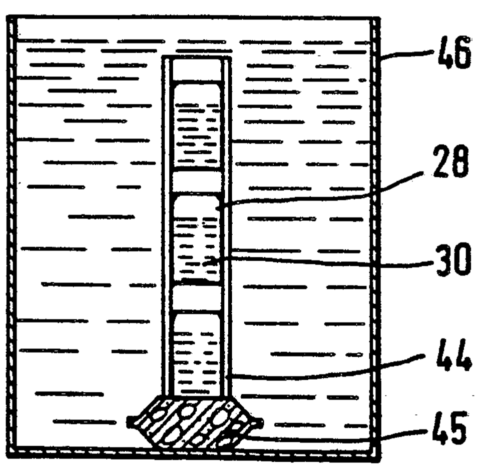


FIG. 11